# EUROPEAN PATENT APPLICATION

(11) **EP 3 320 897 A1**
(43) Date of publication of application: **16.05.2018**
(21) Application number: 16198595.7
(22) Date of filing: 14.11.2016
(51) Int. Cl.: A61K 9/50, A61K 9/16, A61K 36/45

(54) **PROCESS FOR THE PREPARATION OF COATED CRANBERRY GRANULES WITH STABLE PROANTHOCYANIDINE CONTENT**

(71) Applicant: Dompè Primary S.r.l, 20122 Milano (IT)
(72) Inventor: DANI, Marco, 50028 Sambuca Val di Pesa (IT); BENEDETTI, Vanni, 50028 Sambuca Val di Pesa (IT); VELLA, Fulvia, 20134 Milano (IT)
(74) Representative: Pistolesi, Roberto

(57) **Abstract**

The present invention discloses the preparation of coated cranberry granules by fluidized-bed granulation.

Granulation is first carried out with a natural juice, i.e. cranberry, followed by coating with a coating agent selected in the group consisting of: xanthan gum, arabic gum, carboxymethylcellulose (CMC) and cellulose derivates, shellac gum, pectins, polyvinyl-pyrrolidone (PVP), hydroxypropylmethylcellulose (HPMC). Xanthan gum is particularly preferred.

Eventually some probiotic, sweeteners, acidity regulators are optionally added.

## Description

### FIELD OF THE INVENTION

The present invention relates to the field of natural extracts manufacturing and stabilization.

### STATE OF THE ART

Cranberry has been used traditionally for the prevention of Urinary Tract Infections (UTI) and its consumption as a food supplement under different forms, extracts or juices, has diffused very rapidly in the most recent years. It has been postulated that Cranberry (*Vaccinium macrocarpon* Aiton) may play a role in the inhibition of E. coli adhesion to cells of the uroepithelial tract, so as to represent a subsidiary treatment in antibiotic therapy or prophylactic for UTI.

On 15 September 2010, the French Agency for Food, Environmental and Occupational Health & Safety issued an internal request for an assessment of the potential effects of cranberry on community-acquired urinary tract infections. This assessment, conducted jointly with the French Health Products Safety Agency (AFSSAPS) finally concluded that: "Concerning the data published *in vitro* and *ex vivo* data, ANSES considers that the activity of PACs contained in cranberry on *E. coli* adherence to urinary epithelial cells via P fimbriae has been demonstrated.".

However, data have been considered insufficient to conclude that consumption of cranberry has a preventive effect on UTIs.

As a matter of fact, although the EFSA has failed to confirm the "cause - effect" relationship between the prophylactic activity on urinary infection (EFSA Journal 2014; 12(5): 3657) and the regular taking of a Cranberry product (CranMax^{®}), several publications disclose that different cranberry-derived products reduce UTI recurrence and antibiotic consumption in elderly women and to relief urinary symptoms during radiotherapy of prostatic carcinoma.

In the publications reviewed in the two European studies, products derived from different natural extract products have been used. The conclusions in both studies, thus, pointed out the most important problem in determining cranberry bioactivity: the lack of a standardized measurement of the PACs content in the different cranberry products available on the market. Positively, the French report did not raise any safety concern related to the consumption of cranberry by the general population, except for individuals prone to oxalate kidney stones.

In any case, although the properties of cranberry juice and extracts are still debated, it appears now clear that proantocyanidines inhibit E. coli adhesion to cells of the uroepithelial and this in turn, has a prophylactic activity on UTIs.

However, it's still necessary to overcome the technical problems highlighted in the above cited references and more generally, in the field, by adopting:
- a correct dosage of the initial PACs content, which is key and preliminary to any evaluation of bioactivity or clinical claim, as clearly pointed out in the French Agency and European Opinion,
- a manufacturing process that preserves (i.e. from oxidation) the initial PACs content.

WO2012/123491 and US2005/0158381 disclose respectively gastro-protected and effervescent PACs, but do not address these problems. Furthermore, they do not disclose the manufacturing process nor the stability of the PACs content after preparation and/or storage.

### SUMMARY OF THE INVENTION

The present invention is related to a 2-steps fluidized-bed granulation process for the preparation of coated cranberry granules, in which dosed Cranberry extracts, preferably in combination with additional ingredients and/or excipients (i.e. prebiotics such as arabinogalactan and/or anti-caking agent such as silicon dioxide), are first granulated with a natural juice and then coated with a coating agent, wherein the coating agent is preferably xanthan gum.

Additional ingredients and/or excipients are preferably selected among the following: prebiotics such as carbohydrates made out of a short chain of fructose molecules, i.e. fructo-oligosaccharides (FOS) such as arabinogalactan, anti-caking agents selected in the group consisting of: silicon dioxide, calcium silicate, magnesium silicate, talc, calcium phosphate. Furthermore sweeteners, acidity regulators may be added before fluidized-bed granulation.

In the coated granules, the PACs content should be higher than 2% (w/w) and is preferably comprised from 2 to 5%, more preferably about 2.77% and is quite stable during time.

The coated granules can be optionally further admixed with acid-sensitive agents, such as probiotics.

A preferred final composition comprises: arabinogalactan (prebiotic agent: 50-90%, more preferably 60-85%, even more preferably 70-80%), silicon dioxide (anticaking agent: 2-10%, more preferably 3-8%, even more preferably 4-6%), cranberry extracts, cranberry juice and xantan-gum.

### BRIEF DESCRIPTION OF THE DRAWINGS

**Figure 1****.** Chemical structure of cranberry's proanthocyanidins of the A2 and B2 type.
**Figure 2****.** Cranberry extracts coated according to the present invention (A) or commercially available (B).

### DETAILED DESCRIPTION OF THE INVENTION

The present invention relates to the preparation, by a fluidized-bed granulation, of coated cranberry granules with a stable PACs (proanthocyanidines) content, from Cranberry natural extracts (*Vaccinium macrocarpon* Aiton) which are preferably in combination with other ingredients and/or excipients selected in the group consisting of: a prebiotic, such as carbohydrates made out of a short chain of fructose molecules, i.e. fructo-oligosaccharides (FOS) or arabinogalactan and anti-caking agents such as silicon dioxide, calcium silicate, magnesium silicate, talc and calcium phosphate and which are than coated with a coating agent. Furthermore sweeteners and /or acidity regulators may be added before fluidized-bed granulation.

This process takes place in a fluidized-bed granulator and it is based on a first granulation step in which a suitably dosed cranberry extract powder, preferably combined with other ingredients (i.e. arabinogalactan, an anti-caking agent such as silicon dioxide), is granulated by spray-drying technique with an aqueous solution followed by a second step of coating with a coating agent.

This technique allows to obtain coated cranberry extracts granules which can co-exist with acid-sensitive products, i.e. probiotic agents, giving a higher stability and better viability of the probiotic agent for quite a long time.

Furthermore, notably, the fluidized-bed process, which is usually considered unsuitable for compounds prone to oxidation, allows surprisingly, to maintain PACs content unaltered after completion of the process and stable for at least 18 months, more preferably for at least 24 months. Proanthocyanidine have been defined as oligomers or polymers of catechins and epicatechins (most abundant species) and gallocatechin and epigallocatechin gallate (less abundant) and belong to the broad category of anti-oxidant compounds.

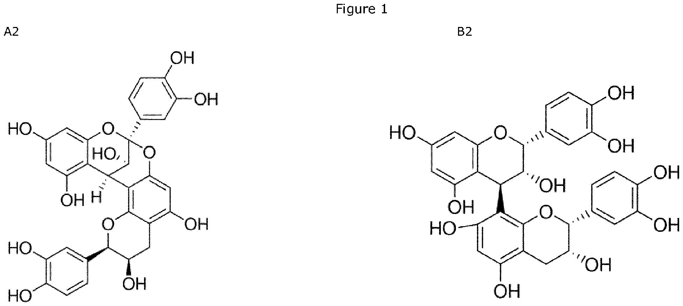

The structure of the most represented monomers in cranberry's proanthocyanidins is shown in figure 1. These monomers, linked respectively by 4β ->6 (or 4β ->8) or 2β ->O ->7 (or 2β ->O ->5) bonds give proanthocyanidines of the B or of the A type. Proanthocyanidines of the A type are most abundant in cranberry (51-65% of the total PACs content) and seem to be mostly responsible for the bacterial anti-adhesion properties (Howell AB et al. Phytochemistry 66:2281-2291 (2005)). Therefore, the first issue to be evaluated when preparing extracts for which a bioactivity is claimed, is the certainty of the source of the cranberry extract. It is necessary, therefore, to use extracts derived from 100% North-American cranberry (*Vaccinium macrocarpon* Aiton); the second critical issue is the certification of the PACs content in the starting material, which must be suitably dosed such so as to provide, in the final form, at least a daily dosage of PACs corresponding to 36 mg/dose. Finally, the third critical issue is to use for the final dosage preparation, a manufacturing process leaving unaltered the initial PACs content of the natural cranberry extracts.

Of course, a certified origin, together with a process which leaves unaltered the initial PACs content is extremely important, since most of the methods commonly used for PACs dosage cannot distinguish among PACs type and PACs deriving from plants from other species such as *Ribes, Vitis,* etc. which are not endowed with the same protective properties, could potentially be used.

As said above, the manufacturing process used for these extracts is critical; proanthocyanidines (PACs) are potent antioxidant molecules which exert their function by becoming substrates for oxidation. Therefore, a certified origin of the starting material is not enough to achieve a good final product, unless the process itself is optimized to maintain the initial PACs content in the natural non-oxidized form.

According to the process of the invention, a suitable mix of certified and selected cranberry extracts (selected and dosed every time before each batch production in order to achieve, once prepared and aliquoted in a daily dose, the suggested PACs content and the required organoleptic characteristics), are preferably combined with other excipients and/or ingredients such as prebiotic agents, preferably arabinogalactan, anti-caking agents preferably silicon dioxide, and then granulated with an aqueous solution, preferably a natural juice or more preferably a diluted cranberry juice.

This juice is usually provided as a concentrate which is diluted with water to a density suitable for being sprayed by the fluid-bed apparatus. Usually, this density is comprised from 15 to 25 °Bx.

The addition of cranberry natural juice has been found to provide an extremely pleasant taste and flavor to the extracts. Furthermore, it provides not only an additional PACs content to the mixture, but also other substances that are believed to enhance, synergistically, their action, such as organic acids typical of the cranberry juice, including quinic acid, but also malic and citric acids.

The organic acids fraction contained in the berries and in the juice, which is by converse not present in the purified extracts of cranberry, is particularly important for the anti-adhesive epithelial action against etiological agents responsible for infections of the urinary tract.

After granulation with the natural juice, a coating agent is sprayed on the granules.

Coating agents are known in the art and are selected in the group consisting of: xanthan gum, arabic gum, carboxymethylcellulose (CMC) and cellulose derivates, shellac gum, pectins, polyvinyl-pyrrolidone (PVP), hydroxypropylmethylcellulose (HPMC). Xanthan gum is particularly preferred: it is preferably added for coating at a concentration of from 0.001 to 1% w/w.

Alternative coating agents are the following: plant gums and/or a plant gelatins. The plant gum and/or the plant gelatin is preferably selected from the group comprising a tannate or a gelatin tannate, an alginate, a xyloglucan or xylogel, a guar gum, a tara gum, an acacia, carob, oat, bamboo fiber, citrus fruit fibers and glucomannans.

After coating, the granulated product is dried for a few minutes at a temperature in which the product temperature never to exceed 45°C. This is achieved by setting the inlet temperature of the fluidized bed granulator in a range of from 50 to 65°C, more preferably of from 55-63°C. However, the setting of the apparatus may be adjusted by the skilled man to achieve the suitable product temperature and to have the product granulated in a few minutes. After the product is cooled off, a probiotic is optionally added, if required. Probiotic agents are preferably those with a documented activity in UTI, which are known to the skilled man and belong preferably to the Lactobacillus species.

Exemplary probiotic agent strains are the following: Lactobacillus plantarum, Lactobacillus paracasei Lactobacillus acidophilus, Lactobacillus Rhamnosus, Lactobacillus Reuteri, Lactobacillus salivarius, Lactobacillus Fermentum and, il Bifidobacterium lactis and Streptococcus Thermophilus. The most preferred is Lactobacillus Paracasei, even more preferably Lpc37. The probiotic is admixed to provide a bacterial load comprised from 1 x10⁸ to 1 x10¹² CFU/g of mixture and to provide a daily dosage of 5 x 10⁹ The composition of the present invention which contains said mixture may has a bacterial load of about 1 x10⁹-1x10¹¹ CFU/composition.

The most preferred is Lactobacillus paracasei Lpc37.

Of note, among the problems that need to be overcome with natural extracts, in particular those derived from cranberry, and which is even more relevant when they have to be mixed with acid-sensitive agents such as probiotics, is their acidity. Cranberry extracts are naturally rich in organic acids, and according to the process of the invention they are first granulated with a natural juice, which is also highly acidic.

Therefore, the coating according to the process, allows these extracts to be associated with probiotics, which are often sensitive to an acidic environment, without altering the probiotic viability and without determining loss of the probiotic load.

In fact this coating acts as a protective barrier on the external surface of the granule, preventing direct contact between acids and probiotics. Furthermore, it also acts as a protective barrier for the PACs in the granule, thus allowing the PACs content to remain stable for at least 18 months, more preferably 24 months and to provide a longer half-life of the final product even when the probiotic component is added. In other words, both the inside PACs content of the natural extract and the external probiotic component are preserved by the coating.

In fact this barrier also avoids the normal processes of oxidation of the PACs caused by the air contained in the pharmaceutical form (envelopes) and so it increases the shelf life of the product, as clearly shown in the experimental part, measured at 18 months and under stress conditions (40°C) (see the Experimental Part).

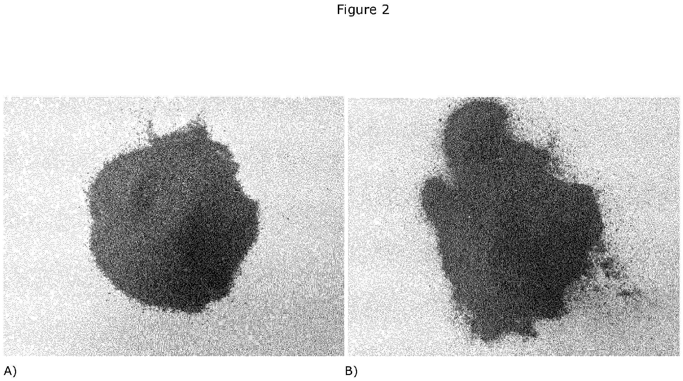

Beside the above mentioned biochemical properties, the coating process carried out after the first granulation with natural juice improves the rheological properties of granules, such as the flowability, decreases significantly the hygroscopicity and thus minimize any problem related to packaging process, which may greatly impair dosage and aliquoting, a problem well known in conventional cranberry extracts, and which impacts the solubility of the final product, in particular after some time on the shelf. An example is shown in figure 2, where the difference between a commercial extract and one obtainable according to the present invention is shown.

In summary, the cranberry extracts granulated and coated according to the process of the present invention show the following properties:
- higher stability
- higher flowability
- higher compatibility upon mixing with acid sensitive components (i.e. probiotics),
and show typically a free water (A_{w}) content not higher than 0.45, preferably comprised from 0.01 to 0.4 and more preferably comprised from 0.05 and 0.3.

### Dosage of PACs

In addition to routine checks needed for compliance with food regulations (such as pesticides, heavy metals, aflatoxins and allergens), a quality control analysis of dry cranberry extracts which are used as raw materials, is usually carried out. The aim of such analysis is to calculate the amount of the (two) extract(s), needed to ensure a final PAC content not below 2% (w/w), more preferably comprised from 2.1 to 4 %, or even more preferably comprised from 2.5% to 3% (i.e. as close as possible to 2.77%) as measured by BL DMAC in the finished granular product (typically a 5.5 grams dose) and to verify the organoleptic characteristics. This analysis is performed for each batch of cranberry extract purchased for the preparation of the finished product.

Several methods are used for the determination of PACs content: the RP-HPLC, the UV based method etc.. The reference method according to the European Pharmacopeia is the Spectrophotometric method (EU Pharm. 6.0; 01/2008:1220); however, recently, due to its validation by the AFSSA (French Food Safety Agency) and to its simplicity and reproducibility, the BL-DMAC colorimetric method has become the most widely used for PACs determination.

The PACs recommended daily dose has been then fixed to 36 mg as determined by the BL-DMAC method.

### Fluidized-bed granulation

This production process is carried out through a fluidized bed granulator. Fluidic-bed granulators, such as IMA GHIBLI 50 and ICF STAR-3, are suitable for this process. These allow the Cranberry Extract to be weighted accurately and to be preferably combined with arabinogalactan and silicon dioxide (raw material), admixed in the drum and then sprayed by an aqueous granulating solution, preferably consisting of diluted cranberry juice.

After granulation, a xanthan gum solution is preferably used for the coating. The process is usually carried out according to the following steps:

### a) Pre-heating of the product

The raw material are introduced into the apparatus drum. This step takes a few minutes, about 5 minutes and allow the product in the drum to reach the ideal temperature for the granulation, which should not be higher than 45°C.

### b) Granulation and preparation of the plant complex

During this stage the powder inside the machine is granulated by spraying an aqueous solution. Preferably the aqueous solution is a Cranberry juice of 65° Bx diluited to about 20°Bx with water. Usually for spraying, the apparatus is set to an inlet air temperature comprised from 75°C to 85°C, more preferably 77°C-83°C and even more preferably of about 80 °C and a flow rate of the pump 220 ml /min).

During the first granulation of the mixture, the rehydrated juice homogeneously granulates the powder. At any stage of the process of formation of the granule, the product temperature is suitably controlled and monitored so as to be ≤45 ° C.

### c) Drying

This phase has a duration a few minutes, it has the aim to ensure the drying of the product at the end of the first phase of granulation. The maximum inlet air temperature at this stage is set not above 55°C. The product temperature should never exceed 45 ° C.

### d) Coating

This stage is required for the coating and finishing of the granule. During this stage of processing the pseudo-granule is coated through the spraying of a xanthan gum aqueous solution (the xanthan gum is 0.06%) useful as a coating agent resistant to acid pH. Even throughout this phase, the product temperature never reaches temperatures >45 ° C.

### e) Drying

This phase has a duration a few minutes, it has the aim to ensure the final drying of the product. The maximum inlet air temperature at this stage is 60°C. The product temperature is ≤45 ° C.

### f) Cooling

The latter stage of processing is required to cool and stabilize the product in order to perform the unloading at a temperature that is as close as possible to room temperature. The phase ends upon reaching the product internal temperature of 25-30 ° C measured with an internal probe. This avoids the absorption of moisture by the product itself.

In order to ensure a dry and stable granule, at the end of the phase of the process, the free water (A_{W}) content and loss on drying of the final product is monitored. Tests on the final product indicate that A_{w} should be in any case below 0.45, preferably comprised from 0.1 to 0.4 and more preferably comprised from 0.05 and 0.3, in order to optimize the stability of the product and improve its shelf-life.

According to a further embodiment the invention refers to the granulated and coated Cranberry extracts, obtainable as a powder with high-flowability by the process according to the invention, and comprising other ingredients and/or excipients, preferably a prebiotic and an anti-caking agent and optionally a probiotic component and showing the following features as compared to commercially available extracts:
- higher stability
- higher flowability
- higher compatibility upon mixing with acid sensitive components (i.e. probiotics). Of note, an improved flowability can be appreciated also from the picture of the product as shown in Figure 2 A.

The following examples of the practice of the present invention are meant to be illustrative and are in no way limiting the scope of the invention.

### EXPERIMENTAL PART

### Example 1. Preparation of the coated cranberry powder

Two certified cranberry extracts were carefully selected, titrated by BL-DMAC as described in Example 3 and mixed in order to obtain a 2.77% w/w PACs content: a) dry Cranberry extract 27-33% BL-DMAC (2.05% w/w), Indena extract; b) dry Cranberry extracts 40% UV (23.59% w/w) (Cranberry extracts, Medtrue Enterprises).

A quantity of about 250 kg of the selected cranberry extract powders was combined with arabinogalactan and silicon dioxide and was then granulated with a fluidized-bed granulator, such as a IMA GHIBLI 50 or ICF STAR-3, as follows: the raw material was weighted accurately, mixed in the drum and then sprayed with a granulating solution, consisting of a diluted cranberry juice (dilution at 20° Brix, from a 65° Brix concentrate), in a quantity of about 12.8 Liters of an aqueous 5.11% v/v in H₂O, at an inlet temperature of 80°C until the formation of granules (i.e. for about 2 hrs in the case of a 250 kg mix). Granules were then coated with a xanthan gum solution 0.06% (0.15 L).

The procedure is better detailed in the following:

### Stage 1: Pre-heating of the product

This process step had a duration of about 5 minutes and serves to bring the product contained in the drum at the ideal temperature for the granulation (45 ° C).

### Stage 2: Granulation and manufacturing of the plant complex.

During this stage the cranberry powder inside the machine was granulated spraying a Cranberry juice 65° Bx suitably diluted to 20°Bx with water (where i.e. a solution at 25 ° Bx contains 25 grams of solid substances in 100 grams of total liquid).

During this phase the first granulation occurs: thanks to pre-established and standardized parameters (inlet air temperature 80 °C and flow rate of the pump 220 ml /min) the dehydrated juice goes to homogeneously coat the powder forming the granule. At any stage of the process of formation of the granule, the product temperature is suitably controlled and monitored so as to never reach values of > 45 ° C.

### Stage 3: Drying

This phase was carried out for a few minutes. It was aimed at ensuring the drying of the product at the end of the first phase of granulation. The maximum inlet air temperature at this stage was 55°C, in order to keep the product temperature below 45°C.

### Stage 4: Coating

During this stage of processing the granule was coated by spraying a xanthan gum aqueous solution 0.06% useful as a coating agent resistant to acid pH.

Even throughout this phase, the product temperature never reaches temperatures >45 ° C. The coating usually takes a few minutes.

### Stage 5: Drying

This phase has a duration a few minutes, it has the aim to ensure the final drying of the product. The maximum inlet air temperature at this stage is 60 ° C. The product temperature is <45°C.

### Stage 6: Cooling

This is the final stage of the process. It is required to cool and stabilize the product. The unloading is performed at a temperature as close as possible to room temperature. The phase ends upon reaching the product internal temperature of 25-30°C, as measured with a temperature probe. This process serves to avoid the absorption of moisture by the product itself.

In order to ensure a dry and stable granule, the free water (A_{W}) and loss on drying of the final product has been measured at the end of this phase of the process. A free water (A_{W}) content of 0.12 was measured.

### Example 2. Mixing of the coated cranberry granules with other ingredients.

Once the coated cranberry granules were obtained, they were admixed with the acid-sensitive components, such as the probiotic, by using an industrial mixer.

The BL-DMAC colorimetric method described in Example 3, was used for the PAC measurement in the starting material and in the final product.

### Example 3. PACs content determination

The BL-DMAC colorimetric method has been disclosed in Prior et al. "Multi-laboratory validation of a standard method for quantifying proanthocyanidins in cranberry powders"; J. Sci Food Agric 2010 (DOI 10.1002/jsfa.3966). Briefly, PACs are first extracted from the samples by an organic solvent solution with acetone (75%), acetic acid (0.5%) and deionized water. Then, a DMAC reagent is added and incubated for 20-30' and OD read at 640nm.

### A commercially available procyanidine A2 (i.e. Sigma Aldrich) is used as analytical standard for the preparation of a calibration curve.

The PACs content has been measured from the coated granules according to the present invention, on a commercial product 2 and a commercially available raw material (commercial extract 1).

Results are shown in the table below.

**Table 1. PACs content measured by different methods**

| Product/extracts | **HPLC TITLE** | **SPECTROPHOTOM. UV - VIS TITLE** | **BL - DMAC TITLE** |
|---|---|---|---|
| Dry cranberry extract (40% UV, Medtrue Ent, Ltd) | 7.30% | 40,32% | 15.00% |
| Dry cranberry extract (20% HPLC, Orocyan ®) | 21.9% | 75.26% | 10.5% |
| Dry cranberry extract 27 - 33% BL - DMAC (Indena) | 7.63% | ND | 27.98% |
| Dry cranberry extract 7% UV (Commercial cranberry extract 1) | 0.72% | 8.33% | ND |
| Mix according to the invention, PACs content 2.77% by BL - DMAC | ND | ND | 2.69 +/-0.164% |
| Commercial product 2 | 0.51% | ND | ND |

| | | | |
|---|---|---|---|
| ND: Not Determined | | | |

The data show the high variability in PACs content, the value of which strongly depends on the method used for the measurement. Furthermore, it shows that is neither possible to see a correspondence nor to define a conversion factor between one measurement and another.

Furthermore, the actual content measured for PACs corresponds only in a few cases to the commercial label of the final product, and this may reflect the important stability issues existing for these products.

### Example 4. Stability tests

The PACs content of the final preparation according to the present invention and one commercial, see commercial product 2, was measured by BL-DMAC, after standard storage conditions 25°C, 65% humidity over a 18 months' time. Under these storage conditions, the PACs content was quite stable for 18 months.

Furthermore, the PACs content was measured also after storage under stress conditions as depicted in the table below and compared to the content measured at time point 0 (corresponding, for the preparation according to the present invention, to time point 0, i.e. immediately after granule coating) or at time point 15 days (i.e. incubated at 40°C for 15 dd after coating) by the BL-DMAC method or the UV spectrophotometric assay. Results are shown in the following table.

**Table 2. Stability tests (PACs content by BL-DMAC and by UV)**

| | T0 PAC conc.% | T15 40°C PAC conc.% | PACs meas. method |
|---|---|---|---|
| Coated granules prepared according to Ex. 1 (present invention) | 2.78% | 2.20% | BL-DMAC |
| " | 16.10 | 14.48 | UV |
| Commercial product 2 | <0.1 | <0.1 | BL-DMAC |
| " | 12.06 | 10,36 | UV |

The content measured after the accelerated decay at 40°C show that the % loss in PACs content is lower for the product prepared according to the present invention; in other words, the treatment according to the present invention leaves the PACs content almost unaltered even in stress condition. Of note, in one of the commercial extracts, the BL-DMAC assay failed to reveal any PACs content.

### Example 5. Dissolution in acidic or basic solutions

3 grams of each sample, a commercial cranberry product (Commercial product 2) and the product prepared according to the present invention were measured for their bioavailability in acidic and neutral pH solutions. The PACs content after 2 hrs stirring was measured by UV.

Both products showed about 90% dissolution at acidic pH and 80% at neutral pH.

## Claims

1. A process for the preparation of coated cranberry granules which comprises a step of fluidized-bed granulation with a natural fruit juice and a step of coating with a coating agent.

2. The process according to claim 1 wherein the natural fruit juice is a cranberry juice.

3. The process according to claim 1 wherein the cranberry extracts are first admixed with other ingredients selected in the group consisting of: prebiotics, excipients, sweeteners, acidity regulators and/or anti-caking agents before fluidized-bed granulation.

4. The process according to any one of claims 1-3 wherein said first granulation is carried out at an inlet temperature of from 75 to 85 °C.

5. The process according to claim 1 wherein said first granulation is preceded by a pre-heating step of the cranberry extracts and optionally the other ingredients, at an inlet temperature comprised from 75-85°C for at least 5 minutes.

6. The process according to claim 1 wherein said granulation is followed by drying the granules at a temperature not higher than 50°C, preferably not higher than 45°C.

7. The process according to any one of claims 1-6 wherein the coating agent is selected in the group consisting of: xanthan gum, arabic gum, carboxymethylcellulose (CMC) and cellulose derivates, shellac gum, pectins, polyvinyl-pyrrolidone (PVP), hydroxypropylmethylcellulose (HPMC).

8. The process according to claim 7 wherein said coating is carried out with an aqueous xantham-gum solution.

9. The process according to any one of claims 1-8 wherein said coating step is followed by a drying step carried out at an inlet temperature not higher than 60°C.

10. The process according to any one of claims 1-9 wherein the granule temperature is kept not higher than 45°C.

11. The process according to claim 9 wherein said drying step is followed by a cooling step, wherein the internal granule temperature is brought below 30°C.

12. The process according to claim 11 which comprises further mixing the coated granules with at least a probiotic agent.

13. Coated cranberry granules obtainable by the process according to claims 1-12 and with a stable cranberry PACs content over 18 months' time.
